# EUROPEAN PATENT SPECIFICATION

(11) **EP 3 246 710 B1**
(45) Date of publication and mention of the grant of the patent: **11.12.2019**
(21) Application number: 15877545.2
(22) Date of filing: 12.06.2015
(51) Int. Cl.: G01N 35/00

(54) **AIR QUALITY DETECTION SYSTEM AND DETECTION METHOD FOR ROBOT**
LUFTQUALITÄTSDETEKTIONSSYSTEM UND DETEKTIONSVERFAHREN FÜR ROBOTER
SYSTÈME DE DÉTECTION DE QUALITÉ D'AIR ET PROCÉDÉ DE DÉTECTION POUR ROBOT

(30) Priority: 12.01.2015 CN 201510015906
(43) Date of publication of application: 22.11.2017
(73) Proprietor: Yutou Technology (Hangzhou) Co., Ltd., Hangzhou City, Zhejiang 311199 (CN)
(72) Inventor: CAI, Mingjun, Hangzhou City Zhejiang 311199 (CN); LIU, Xin, Hangzhou City Zhejiang 311199 (CN)
(74) Representative: Zoli, Filippo
(86) International application number: PCT/CN2015/081401
(87) International publication number: WO 2016/112629

(56) References cited:
- CN-A- 101 162 233
- CN-A- 102 854 849
- CN-A- 102 854 849
- CN-U- 202 649 217
- JP-A- H07 134 617
- US-A1- 2006 293 789
- US-A1- 2006 293 789
- US-A1- 2010 030 382
- DATABASE WPI Week 201379 Thomson Scientific, London, GB; AN 2013-W14940 XP002784235, & CN 203 178 799 U (UNIV HEBEI TECHNOLOGY) 4 September 2013 (2013-09-04)
- DATABASE WPI Week 201010 Thomson Scientific, London, GB; AN 2010-A70169 XP002784236, & CN 201 376 667 Y (ZHANG J) 6 January 2010 (2010-01-06)
- DATABASE WPI Week 200871 Thomson Scientific, London, GB; AN 2008-M04540 XP002784237, & CN 201 107 319 Y (UNIV SOUTH CHINA TECHNOLOGY) 27 August 2008 (2008-08-27)

## Description

### BACKGROUND OF THE INVENTION

### 1. Field of the Invention

The present disclosure relates to the field of robotics, more specifically, to a detecting system and a method for air's quality based on a robot.

### 2. Description of the Related Art

Nowadays the environment changes rapidly, the air's quality fluctuates according to plant emissions, automobile exhaust, and exhaust gas. Increased air pollution is linked to an escalation in the incidences of diseases, such as asthma, lung cancer, cardiovascular disease, birth defects, and premature death. It is important to monitor the air's quality in our living and working environments, and to maintain purified air. Although air detecting devices and air detecting systems have been provided in prior art, those air detecting device and the air detecting system can only be used to detect a single air component, for example, it can only detect the concentration of carbon dioxide, or it can only detect the concentration of another specific harmful gas present in the air. Therefore, because the detecting method is particular, it cannot present the status of the present physical environment fully. Meanwhile, neither the air detecting devices nor the air detecting systems can execute necessary operations, such as purifying the air, which is relative to the present environment, so that the present environment is consistent. Known robot detection systems are disclosed in CN203178799U, CN102854849 and US20060293789.

### SUMMARY OF THE INVENTION

To resolve the shortcoming of the prior art, the invention provides a detecting system in accordance with independent claim 1 as well as a detection method for air quality in accordance with independent claim 5, which adjusts the status of air with various detecting types.

The technical scheme of the invention is:
A detecting system for air quality comprising:
a collection unit configured in a preset position on a robot, which is used to detect the air's condition in immediate surroundings of the robot, and then to form an output of collection data;
a processing unit connected to the collection unit, which is configured to receive the collection data and simultaneously receive the storage data which is output by a storage unit and is matched to the collection data, and then to form a processing result in accordance with the collection data and the storage data, as well as to simultaneously output a control signal;
a storage unit connected to the processing unit, which is configured to store the processing result;
an execution unit, whose control end is connected to the processing unit by a communication module to execute, under the effect of the control signal, actions that match the control signal;
an input/output device connected to the processing unit, which is used to receive and output the processing result, or to receive control instructions input by users, and then to form a control signal and transmit it to the processing unit.

The collection unit comprises a temperature sensor, which is configured to collect the temperature information of the immediate surroundings of the robot, and then to form an output of temperature signals.

Preferably, the collection unit comprises a humidity sensor, which is configured to collect the humidity information of the immediate surroundings of the robot, and then to form an output of humidity signals.

Preferably, the collection unit comprises a carbon dioxide sensor, which is configured to collect the carbon dioxide concentration information of the immediate surroundings of the robot, and then to form an output of carbon dioxide concentration signals.

Preferably, the collection unit comprises a dust sensor, which is configured to collect the dust concentration information of the immediate surroundings of the robot, and then to form an output of dust concentration signals.

The collection unit comprises a gas sensor, which is configured to collect the control gas composition, concentration information of the immediate surroundings of the robot, and then to form an output of gas signals.

A detecting method for air quality wherein it comprises of the following steps:
(a) controlling a collection unit to detect the air's condition in the immediate surroundings of the robot, and to form an output of collection data;
(b) controlling a processing unit to receive the collection data and simultaneously receive the storage data that is output by a storage unit and is matched to the collection data, and to form a processing result in accordance with the collection data and the storage data;
(c) controlling an input/output device to receive the processing result and output the corresponding processing result.

Preferably, it further comprises the following steps:
(d) controlling the input/output device to receive control instructions input by the user, and to form a control signal and transmit it to the processing unit;
(e) making the processing unit to receive the control signal, and then forming the processing result in accordance with the control signal, the processing result is output; and execute step (c).

The benefit of the invention comparative to the prior art is, by adopting the above technical solution, is that it is more convenient for the user to know the air's status information of present environment in real time, and easier to make an necessary adjustments which is beneficial to the health of the user.

### BRIEF DESCRIPTIONS OF THE DRAWINGS

Figure 1 shows the structure diagram of the invention;
Figure 2 shows the process schematic diagram of the invention.

### DETAILED DESCRIPTIONS

Hereinafter, certain exemplary embodiments, according to the present invention, will be described with reference to the accompanying drawings. This does not limit the scope of the invention.

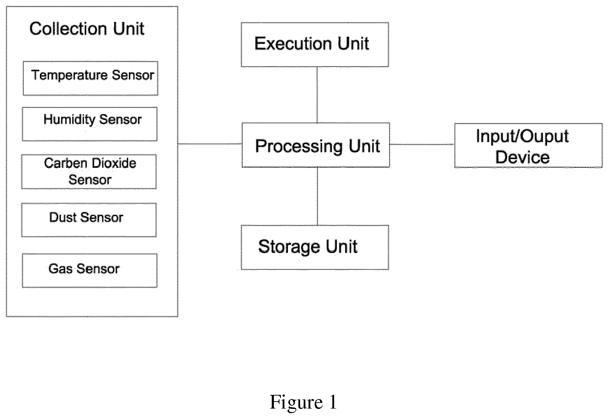

Referring to Figure 1, the detecting system for the air quality comprises:
a collection unit configured in a preset position on a robot, which is used to detect the air condition in the immediate surroundings of the robot, and then to form an output of collection data;
a processing unit connected to the collection unit, which is configured to receive the collection data and simultaneously receive the storage data, which is output by a storage unit, which is matched to the collection data, as well as to form a processing result in accordance with the collection data and the storage data; a storage unit connected to the processing unit, configured to store the processing result;
an execution unit, whose control end is connected to the processing unit by a communication module to execute, under the effect of the control signal, actions that match the control signal; the execution unit can be a temperature adjustment unit, an air purification unit, a humidity adjustment unit, or an air exhaust adjustment unit;
an input/output device connected to the processing unit, which is used receive and output the processing result, as well as to receive control instructions input by the user, and to form a control signal and transmit it to the processing unit.

The working principle of the invention is the following: the collection unit collects information on the air condition of the immediate surroundings of the robot, and then forms collection data and outputs it to the processing unit; the processing unit comprehensively analyzes and processes the collection data in accordance with the collection date and the existing processing result stored in the storage unit, which is used to form and output the processing result of the air conditions in the immediate surroundings; the input/output device can be a voice playing device and/or a display device configured to play and/or display the processing result; the execution unit executes the corresponding operations and improves the air's quality in the immediate surroundings. The input/output device can also receive the control instruction input by the user, and transform the control instruction to one that can be identified by the processing unit, under the effect of which, a processing result is formed and output to the output/input device, the output/input device then displays the processing result. By adopting the above technical solution, it facilitates the user to know the information regarding the air's condition within the immediate surroundings in real time, and it automatically makes necessary adjustments, which is beneficial to the health of the user.

In accordance with the invention, the collection unit comprises:
a temperature sensor, which is configured to collect the temperature information of the immediate surroundings of the robot, as well as to form an output of temperature signal. If the environment temperature is too low or too high, the processing unit sends out the control command, according to the present temperature signal, to adjust the temperature adjustment device, to maintain the temperatuer at a the level which people feel comfortable. In a further preferred embodiment the temperature adjustment device can further perform as an air conditioner, a receiver end of the air condition control unit is connected to the processing unit, the receiver end is configured to receive the remote control command sent from the processing unit, and the remote control commands are matched to the air condition; the control unit then executes the corresponding operation according to the remote control command, such as sending hot airflow or sending cold airflow.

In accordance with the invention when the indoor temperature ascends continually and exceeds the threshold preset by the processing unit, the processing unit determines the heat source and sends a control signal to the sprinkler, the sprinkler is then initiated; the sprinkler then sprays liquild at the heat source to cool down it, so to avoid the fire hazard.

In a further preferred embodiment, the collection unit comprises a humidity sensor, which is configured to collect the humidity information of the immediate surroundings of the robot, and then to form an output of the humidity signal. The processing unit sends out the control command, according to the present humidity signal, to adjust the humidity adjustment device, which maintains the humidity at a level that makes people feel comfortable. The humidity adjustment device can further perform as a humidifier, a control end of the humidifier is connected to the processing unit, the control end outputs corresponding moisture under the effect of the control command, to adjust the indoor temperature. The humidity adjustment device can also be an air conditioner.

In a further preferred embodiment, the collection unit comprises a carbon dioxide sensor configured to collect the carbon dioxide concentration information of the immediate surroundings of the robot, it then form an output signal of the carbon dioxide concentration. The control unit sends out a control command according to the present carbon dioxide concentration signal, which is used to adjust ventilation, thus adjusting the present carbon dioxide concentration. The ventilation unit can be a ventilating fan. The control end of the ventilating fan is connected to the processing unit; the ventilating fan is initiated under the effect of the control command, so that the ventilation is accelerated, and the carbon dioxide concentration is decreased.

In a further preferred embodiment, the collection unit comprises a dust sensor, which is configured to collect the dust concentration information of the immediate surroundings of the robot, and then to form an output of the dust concentration signal. The control unit sends out control command according to the present dust concentration signal to adjust the air purification unit, which adjusts the dust concentration in the air. The air purification unit can further perform as an air cleaner. The control end of the air cleaner is connected to the processing unit; the air cleaner is initiated under the effect of the control command to purify the indoor air, and to improve the indoor air quality. The purification unit can also be formed by an exhaust system of an air condition.

In accordance with the invention the collection unit comprises a gas sensor, which is configured to collect the control gas composition and concentration information of the immediate surroundings of the robot, and then it forms an output of the gas signal. The gas sensor is mainly used for detecting any harmful gas concentration in the air. The processing unit sends out control commands, according to the present gas composition and concentration information, which is used to adjust the air purification unit, then, in turn adjusts the harmful gas in the air. The harmful gas includes but is not limited to methanal, volatile organic compounds, etc. The air purification unit can further perform as an air cleaner.

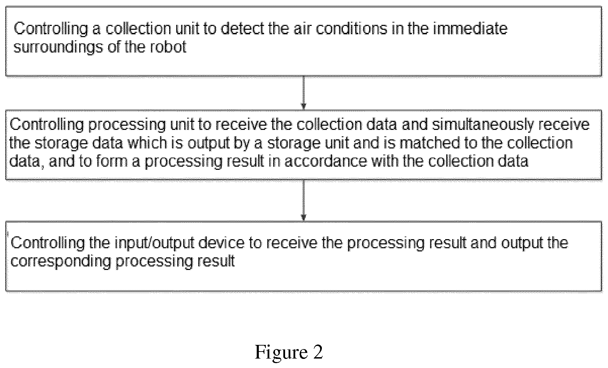

Referring to Figure 2, a detecting method for air quality, comprises the following steps:
(a) controlling a collection unit to detect the air conditions in the immediate surroundings of a robot, and then to form an output of collection data;
(b) controlling processing unit to receive the collection data and simultaneously receive the storage data which is output by a storage unit and is matched to the collection data, and to form a processing result in accordance with the collection data;
(c) controlling the input/output device to receive the processing result and output the corresponding processing result.

In a further preferred embodiment, it also comprises the following steps:
(d) controlling the input/output device to receive the control instructions input by the user, and to form a control signal and transmit it to the processing unit;
(e) making the processing unit receive the control signal, and then forming the processing result in accordance with the control signal, the processing result is output; and execute step (c).

The working principle of the detection method for the air's quality, based on robot, has been described in the above detection system clearly, and it will not be described any more.

The above description is only the preferred embodiments of the invention, and it does not limit the implement method and protecting scope of the invention. It is obvious for those skilled in the art that the changes and variations made by the specification and drawings of the invention should fall within the scope of the invention.

## Claims

1. An air quality detecting system comprising:
a collection unit configured in a preset position on a robot, which is used to detect the air condition in immediate surroundings of the robot, and then to form an output of collection data;
a processing unit connected to the collection unit, which is configured to receive the collection data and simultaneously receive the storage data which is output by a storage unit and is matched to the collection data, and then to form a processing result in accordance with the collection data and the storage data, as well as to simultaneously output a control signal;
a storage unit connected to the processing unit, which is configured to store the processing result;
an execution unit, whose control end is connected to the processing unit by a communication module to execute, under the effect of the control signal, actions that match the control signal;
an input/output device connected to the processing unit, which is used to receive and output the processing result, or to receive control instructions input by users, and then to form a control signal and transmit it to the processing unit;
the collection unit comprises a temperature sensor, which is configured to collect the temperature information of the immediate surroundings of the robot, and then to form an output of temperature signals;
if the environment temperature is too low or too high, the processing unit sends out a control command, according to present temperature signal, to adjust a temperature adjustment device, to maintain the temperature at a level which people feel comfortable; when indoor temperature ascends continually and exceeds threshold preset by the processing unit, the processing unit determines the heat source and sends a control signal to a sprinkler, the sprinkler is then initiated; the sprinkler then sprays liquid at the heat source to cool it down,
the collection unit comprises a gas sensor, which is configured to collect gas composition, concentration information of immediate surroundings of the robot, and then to form an output of gas signals, the processing unit sends out control commands, according to present gas composition and concentration information, which is used to adjust an air purification unit, then, in turn adjusts harmful gas in the air.

2. The detecting system for air quality as claimed in claim 1, wherein the collection unit comprises a humidity sensor, which is configured to collect the humidity information of the immediate surroundings of the robot, and then to form an output of humidity signals.

3. The detecting system for air quality as claimed in claim 1, wherein the collection unit comprises a carbon dioxide sensor, which is configured to collect the carbon dioxide concentration information of the immediate surroundings of the robot, and then to form an output of carbon dioxide concentration signals.

4. The detecting system for air quality as claimed in claim 1, wherein the collection unit comprises a dust sensor, which is configured to collect the dust concentration information of the immediate surroundings of the robot, and then to form an output of dust concentration signals.

5. An air quality detecting method using a system in accordance with any of the claims 1-4, wherein the method comprises the following steps:
(a) controlling a collection unit to detect the air condition in the immediate surroundings of the robot, and to form an output of collection data; the collection data includes temperature information of the immediate surroundings of the robot, the collection data also includes gas composition, concentration information of immediate surroundings of the robot;
(b) controlling a processing unit to receive the collection data and simultaneously receive the storage data that is output by a storage unit and is matched to the collection data, and to form a processing result in accordance with the collection data and the storage data;
(c) controlling an input/output device to receive the processing result and output the corresponding processing result;
(d) controlling the input/output device to receive control instructions input by the user, and to form a control signal and transmit it to the processing unit;
(e) making the processing unit to receive the control signal, and
then forming the processing result in accordance with the control signal, the processing result is output; and execute step (c);
if the environment temperature is too low or too high, the processing unit sends out a control command, according to present temperature signal, to adjust temperature adjustment device, to maintain the temperature at a level which people feel comfortable; when indoor temperature ascends continually and exceeds threshold preset by the processing unit, the processing unit determines heat source and sends a control signal to sprinkler, the sprinkler is then initiated; the sprinkler then sprays liquid at the heat source to cool down it,
the processing unit sends out control commands, according to present gas composition and concentration information, which is used to adjust an air purification unit, then, in turn adjusts harmful gas in the air.

## Patentansprüche

1. Luftqualitätserfassungssystem, umfassend:
eine Sammeleinheit, die an einer voreingestellten Position an einem Roboter angeordnet ist, welche verwendet wird, um die Luftbedingungen in der unmittelbaren Umgebung des Roboters zu erfassen und anschließend eine Ausgabe der gesammelten Daten zu erzeugen;
eine mit der Sammeleinheit verbundene Verarbeitungseinheit, die dafür konfiguriert ist, die gesammelten Daten zu empfangen und gleichzeitig die von einer Speichereinheit ausgegebenen und an die gesammelten Daten angepassten gespeicherten Daten zu empfangen, und anschließend ein Verarbeitungsergebnis entsprechend der gesammelten Daten und der gespeicherten Daten zu erzeugen und gleichzeitig ein Steuersignal auszugeben;
eine mit der Verarbeitungseinheit verbundene Speichereinheit, die für das Speichern des Verarbeitungsergebnisses konfiguriert ist;
eine Ausführungseinheit, deren Steuerende durch ein Kommunikationsmodul mit der Verarbeitungseinheit verbunden ist, um unter der Wirkung des Steuersignals Aktionen auszuführen, die mit dem Steuersignal übereinstimmen;
eine mit der Verarbeitungseinheit verbundene Eingabe-/Ausgabevorrichtung, die dazu dient, das Verarbeitungsergebnis zu empfangen und auszugeben oder von Benutzern eingegebene Steueranweisungen zu empfangen und anschließend ein Steuersignal zu erzeugen und an die Verarbeitungseinheit zu senden;
die Sammeleinheit umfasst einen Temperatursensor, der dafür konfiguriert ist, die Temperaturinformationen der unmittelbaren Umgebung des Roboters zu sammeln und anschließend eine Ausgabe von Temperatursignalen zu erzeugen;
wenn die Umgebungstemperatur zu niedrig oder zu hoch ist, sendet die Verarbeitungseinheit einen Steuerbefehl gemäß dem vorliegenden Temperatursignal, um eine Vorrichtung zur Temperatureinstellung einzustellen, um die Temperatur auf einem Niveau zu halten, auf dem sich Menschen wohl fühlen;
wenn die Innentemperatur kontinuierlich ansteigt und den von der Verarbeitungseinheit vorgegebenen Schwellenwert überschreitet, bestimmt die Verarbeitungseinheit die Wärmequelle und sendet ein Steuersignal an einen Sprinkler, wobei der Sprinkler anschließend gestartet wird;
der Sprinkler sprüht anschließend Flüssigkeit auf die Wärmequelle, um diese abzukühlen, wobei die Sammeleinheit einen Gassensor umfasst, der dafür konfiguriert ist, die Gaszusammensetzung, Konzentrationsinformationen der unmittelbaren Umgebung des Roboters zu sammeln und anschließend eine Ausgabe von Gassignalen zu erzeugen, wobei die Verarbeitungseinheit Steuerbefehle gemäß der vorliegenden Gaszusammensetzung und Konzentrationsinformation sendet, die zum Einstellen einer Luftreinigungseinheit verwendet werden, und anschließend wiederum schädliches Gas in der Luft einstellt.

2. Luftqualitätserfassungssystem nach Anspruch 1, wobei die Sammeleinheit einen Feuchtigkeitssensor umfasst, der dafür konfiguriert ist, die Feuchtigkeitsinformationen der unmittelbaren Umgebung des Roboters zu sammeln und dann eine Ausgabe von Feuchtigkeitssignalen zu erzeugen.

3. Luftqualitätserfassungssystem nach Anspruch 1, wobei die Sammeleinheit einen Kohlendioxidsensor umfasst, der dafür konfiguriert ist, die Kohlendioxidkonzentrations-Informationen der unmittelbaren Umgebung des Roboters zu sammeln und dann eine Ausgabe von Kohlendioxidkonzentrations-Signalen zu erzeugen.

4. Luftqualitätserfassungssystem nach Anspruch 1, wobei die Sammeleinheit einen Staubsensor umfasst, der konfiguriert ist, um die Staubkonzentrations-Informationen der unmittelbaren Umgebung des Roboters zu sammeln und dann eine Ausgabe von Staubkonzentrations-Signalen zu erzeugen.

5. Luftqualitätserfassungsverfahren unter Verwendung eines Systems nach einem der Ansprüche 1 bis 4, wobei das Verfahren die folgenden Schritte umfasst:
(a) Steuern einer Sammeleinheit, um die Luftbedingungen in der unmittelbaren Umgebung des Roboters zu erfassen und eine Ausgabe von gesammelten Daten zu erzeugen;
die gesammelten Daten beinhalten Temperaturinformationen der unmittelbaren Umgebung des Roboters, wobei die gesammelten Daten außerdem Daten zur Gaszusammensetzung sowie Konzentrationsinformationen der unmittelbaren Umgebung des Roboters enthalten;
(b) Steuern eine Verarbeitungseinheit, um die gesammelten Daten zu empfangen und gleichzeitig die von einer Speichereinheit ausgegebenen und an die gesammelten Daten angepassten gespeicherten Daten zu empfangen, und um ein Verarbeitungsergebnis entsprechend der gesammelten Daten und der gespeicherten Daten zu erzeugen;
(c) Steuern einer Eingabe-/Ausgabevorrichtung, um das Verarbeitungsergebnis zu empfangen und das entsprechende Verarbeitungsergebnis auszugeben;
(d) Steuern der Eingabe-/Ausgabevorrichtung, um vom Benutzer eingegebene Steuerbefehle zu empfangen, und um ein Steuersignal zu erzeugen und es an die Verarbeitungseinheit zu übertragen;
(e) Veranlassen, dass die Verarbeitungseinheit das Steuersignal empfängt, und anschließend das Verarbeitungsergebnis in Übereinstimmung mit dem Steuersignal erzeugt, wobei das Verarbeitungsergebnis ausgegeben wird; und Ausführen von Schritt (c);
wenn die Umgebungstemperatur zu niedrig oder zu hoch ist, sendet die Verarbeitungseinheit einen Steuerbefehl gemäß dem vorliegenden Temperatursignal, um eine Vorrichtung zur Temperatureinstellung einzustellen, um die Temperatur auf einem Niveau zu halten, auf dem sich Menschen wohl fühlen;
wenn die Innentemperatur kontinuierlich ansteigt und den von der Verarbeitungseinheit vorgegebenen Schwellenwert überschreitet, bestimmt die Verarbeitungseinheit die Wärmequelle und sendet ein Steuersignal an einen Sprinkler, wobei der Sprinkler anschließend gestartet wird;
der Sprinkler sprüht anschließend Flüssigkeit auf die Wärmequelle, um diese abzukühlen, wobei die Verarbeitungseinheit Steuerbefehle gemäß der vorliegenden Gaszusammensetzung und Konzentrationsinformation sendet, die zum Einstellen einer Luftreinigungseinheit verwendet werden, und anschließend wiederum schädliches Gas in der Luft einstellt.

## Revendications

1. Système de détection de la qualité de l'air comprenant :
une unité de collecte configurée dans une position prédéfinie sur un robot, qui est utilisée pour détecter l'état de l'air dans les environs immédiats du robot, et pour former ensuite une sortie de données de collecte ;
une unité de traitement reliée à l'unité de collecte, qui est configurée pour recevoir les données de collecte et pour recevoir simultanément les données de stockage qui sont délivrées en sortie par une unité de stockage et sont mises en correspondance avec les données de collecte, et pour former ensuite un résultat de traitement en fonction des données de collecte et des données de stockage, ainsi que pour délivrer en sortie simultanément un signal de commande ;
une unité de stockage reliée à l'unité de traitement, qui est configurée pour stocker le résultat de traitement ;
une unité d'exécution, dont l'extrémité de commande est reliée à l'unité de traitement par un module de communication pour exécuter, sous l'effet du signal de commande, des actions qui correspondent au signal de commande ;
un dispositif d'entrée/sortie relié à l'unité de traitement, qui est utilisé pour recevoir et pour délivrer en sortie le résultat de traitement, ou pour recevoir des instructions de commande entrées par des utilisateurs, et pour former ensuite un signal de commande et le transmettre à l'unité de traitement ;
l'unité de collecte comprend un capteur de température, qui est configuré pour collecter les informations de température des environs immédiats du robot, et pour former ensuite une sortie de signaux de température ;
si la température ambiante est trop basse ou trop élevée, l'unité de traitement envoie un ordre de commande, en fonction d'un signal de température actuelle, pour régler un dispositif de réglage de température, afin de maintenir la température à un niveau auquel les gens se sentent à l'aise ; lorsque la température intérieure augmente continuellement et dépasse un seuil prédéfini par l'unité de traitement, l'unité de traitement détermine la source de chaleur et envoie un signal de commande à un pulvérisateur, le pulvérisateur est alors amorcé ;
le pulvérisateur vaporise ensuite un liquide au niveau de la source de chaleur pour la refroidir,
l'unité de collecte comprend un capteur de gaz, qui est configuré pour collecter des informations de composition, de concentration de gaz des environs immédiats du robot, et pour former ensuite une sortie de signaux de gaz, l'unité de traitement envoie des ordres de commande, en fonction des informations de composition et de concentration actuelles de gaz, qui sont utilisés pour régler une unité de purification de l'air, puis, à son tour, règle les gaz nocifs dans l'air.

2. Système de détection de la qualité de l'air selon la revendication 1, dans lequel l'unité de collecte comprend un capteur d'humidité, qui est configuré pour collecter les informations d'humidité des environs immédiats du robot, et pour former ensuite une sortie de signaux d'humidité.

3. Système de détection de la qualité de l'air selon la revendication 1, dans lequel l'unité de collecte comprend un capteur de dioxyde de carbone, qui est configuré pour collecter les informations de concentration de dioxyde de carbone des environs immédiats du robot, et pour former ensuite une sortie de signaux de concentration de dioxyde de carbone.

4. Système de détection de la qualité de l'air selon la revendication 1, dans lequel l'unité de collecte comprend un capteur de poussière, qui est configuré pour collecter les informations de concentration de poussière des environs immédiats du robot, et pour former ensuite une sortie de signaux de concentration de poussière.

5. Procédé de détection de la qualité de l'air utilisant un système selon l'une des revendications 1 à 4, dans lequel le procédé comprend les étapes suivantes consistant à :
(a) commander une unité de collecte pour détecter l'état de l'air dans les environs immédiats du robot et pour former une sortie des données de collecte ; les données de collecte incluent des informations de température des environs immédiats du robot, les données de collecte incluent également des informations de composition et de concentration de gaz des environs immédiats du robot ;
(b) commander une unité de traitement pour recevoir les données de collecte et pour recevoir simultanément les données de stockage qui sont délivrées en sortie par une unité de stockage et sont mises en correspondance avec les données de collecte, et à former un résultat de traitement en fonction des données de collecte et des données de stockage ;
(c) commander un dispositif d'entrée/sortie pour recevoir le résultat de traitement et pour délivrer en sortie le résultat de traitement correspondant ;
(d) commander le dispositif d'entrée/sortie pour recevoir des instructions de commande saisies par l'utilisateur, et à former un signal de commande et le transmettre à l'unité de traitement ;
(e) faire en sorte que l'unité de traitement reçoive le signal de commande, et à former ensuite le résultat de traitement en fonction du signal de commande, le résultat de traitement est délivré en sortie ; et à exécuter l'étape (c) ;
si la température ambiante est trop basse ou trop élevée, l'unité de traitement envoie un ordre de commande, en fonction d'un signal de température actuelle, pour régler un dispositif de réglage de température, afin de maintenir la température à un niveau auquel les gens se sentent à l'aise ; lorsque la température intérieure augmente continuellement et dépasse un seuil prédéfini par l'unité de traitement, l'unité de traitement détermine une source de chaleur et envoie un signal de commande à un pulvérisateur, le pulvérisateur est alors amorcé ;
le pulvérisateur vaporise ensuite un liquide au niveau de la source de chaleur pour la refroidir,
l'unité de traitement envoie des ordres de commande, en fonction des informations de composition et de concentration actuelles de gaz, qui sont utilisés pour régler une unité de purification de l'air, puis, à son tour, règle les gaz nocifs dans l'air.
